# EUROPEAN PATENT SPECIFICATION

(11) **EP 1 883 392 B1**
(45) Date of publication and mention of the grant of the patent: **22.12.2010**
(21) Application number: 06770886.7
(22) Date of filing: 22.05.2006
(51) Int. Cl.: A61K 9/10, A61P 23/02

(54) **PROCESS FOR PREPARATION OF ISOTONIC AQUEOUS INJECTION OF ROPIVACAINE**
VERFAHREN FÜR DIE ZUBEREITUNG EINER ISOTONISCHEN WÄSSRIGEN ROPIVACAIN-INJEKTION
PROCEDE DE PREPARATION D'UNE INJECTION AQUEUSE ISOTONIQUE DE ROPIVACAINE

(30) Priority: 25.05.2005 US 137256
(43) Date of publication of application: 06.02.2008
(73) Proprietor: Navinta, llc., Ewing, NJ 08618-1414 (US)
(72) Inventor: JOBDEVAIRAKKAM, Christopher N., Ewing, NJ 08618 (US); RANGISETTY, Jagadeesh B., Ewing, NJ 08618 (US)
(74) Representative: Henriksen, Michael
(86) International application number: PCT/US2006/019804
(87) International publication number: WO 2006/127639

(56) References cited:
- US-A1- 4 695 576
- US-A1- 4 870 086

## Description

### Field of the invention

The present invention relates to a process for the preparation of an injectable, preferably isotonic, solution of ropivacaine, with optional adjustment of the pH and/or osmolality of the solution, and without the need for a hydrochloride or hydrate intermediate.

### Background of the invention

Ropivacaine is the generic name of the n-propyl homolog of the recently introduced long active local anesthetics having the general formula N-(n-alkyl)-2,6-dimethylpheny-piperidine-2-carboxamide. Optically pure ropivacaine is the levo form, (2S)-N-(n-propyl)-2,6-dimethylphenyl-piperidine-2-carboxamide; another chemical name for ropivacaine is (L) N-n-propylpipecolic acid-2,6-xylidide. The optically pure form of ropivacaine is reported to have reduced cardio-toxic potential compared to the racemic mixture of bupivacaine (racemic N-n-butylpipecolic acid-2,6-xylidide, having better analgesic effects than either D or L isomer alone, as described in US patent 4,695,576); it has been suggested that an alkyl group of five carbons is too toxic for practical anesthetic use. (All of the patents referenced herein are incorporated by reference in their entirety.)

The preparation and purification of optically pure ropivacaine and its salts has been disclosed in the art. WO 85/00599 and US Pat. Nos. US 4,695,576 and US 4,870,086 describe the preparation of (L) N-n-propylpipecolic acid-2,6-xylidide and its water soluble salts.

As described in the following art, the state of the art is that injectable solutions are made from a salt (e.g., hydrochloride) or a hydrate (e.g., monohydrate hydrochloride).

The aforementioned US 4,695,576 describes the optically pure compound (L) N-n-propylpipecolic acid-2,6-xylidide in the form of monohydrate hydrochloride. This patent discloses a process for the preparation of (L) N-n-propylpipecolic acid-2,6-xylidide hydrochloride and also discloses that (L) N-n-propylpipecolic acid-2,6-xylidide may be used as an injectable local anesthetic in the form of water soluble salt. However, preparing an aqueous solution of (L) N-n-propylpipecolic acid-2,6-xylidide directly is not possible due to solubility limitations.

US Pat. No. 4,870,086 reports an observation that the (L) N-n-propylpipecolic acid-2,6-xylidide hydrochloride prepared as described in WO 85/00599 is hygroscopic and thus not stable, leading to the invention of (L) N-n-propylpipecolic acid-2,6-xylidide hydrochloride monohydrate prepared from the hydrochloride. This patent also discloses the use of the hydrochloride monohydrate in the preparation of pharmaceutical preparations by dissolving the hydrochloride monohydrate in a liquid diluent suitable for injection. The example given describes dissolving the hydrochloride monohydrate in sterile water, adding sodium chloride, and then adjusting the pH with sodium hydroxide.

US patent US 4,870,086 also mentions that the monohydrochloride of ropivacaine is hygroscopic and thus not stable. As made in the '086 patent, the monohydrochloride contains 2% of water, and the hydrochloride monohydrate salt of ropivacaine contains about 5.5 % water. Only on heating the hydrochloride monohydrate at 75 °C. for 16 hours is the water removed. Practically, therefore, it will be difficult to dry the ropivacaine hydrochloride monohydrate at the commercial manufacturing scale to remove all the solvent used in the process without also losing water.

US Pat. No. 5,932,597 describes a process of preparing an injectable formulation of 1-alkyl-N-(2,6-dimethylphenyl)-2-piperidinecarboxamide in the presence of a saccharide, specifically glucose, with an example provided for levobupivacaine; the source of levobupivacaine in the production of the injectable formulation is from its hydrochloride salt.

As described in the foregoing literature, the injectable solution of (L) N-n-propylpipecolic acid-2,6-xylidide is prepared from its hydrochloride or hydrochloride monohydrate salts, which are easily soluble in injectable media, such as aqueous solution.

US 4,695,576 mentions the use of (L) N-n-propylpipecolic acid-2,6-xylidide base in suppositories or topical anesthetic by being blended with conventional solvents and carriers including thixotropic mixtures which form gels, in a suspension, or tablet by using conventional materials. Also disclosed is the preparation of aqueous injectables, but, again, only from the salts.

### Summary of the Invention

According to an aspect of the present invention there is provided a process for making a solution of ropivacaine base in an aqueous medium as claimed in Claim 1.

Nowhere in the literature is an injectable anesthetic preparation of water insoluble xylidide base described where only inorganic (mineral) acid and xylidide base are used without a hydrochloride or hydrate intermediate.

Accordingly, one object of this invention is to provide a method for making an aqueous injectable ropivacaine base using only inorganic acid and the base without a hydrochloride or hydrochloride monohydrate intermediate.

Another object of this invention is to provide such a method where the aqueous injectable ropivacaine base is isotonic.

Still a further object of this invention is to provide such a method where the aqueous injectable ropivacaine base has a desired osmolality.

In summary, this invention discloses the preparation of an injectable solution of ropivacaine by dissolving ropivacaine base in aqueous acidic solution having a molar ratio of acid to ropivacaine base greater than 1.1 eliminates the need for a hydrochloride or hydrochloride monohydrate intermediate in the manufacture of the injectable. The osmolality can be adjusted as needed. The excess acid is neutralized with a base. More particularly, the novel method of this invention for preparing an injectable solution of (L) N-n-propylpipecolic acid-2,6-xylidide, hereinafter referred as ropivacaine base, is by dissolving the ropivacaine base in a suitable aqueous medium acceptable for injection and having an excess of a pharmaceutically acceptable acid, optionally adding sodium chloride to adjust the osmolality, and then adjusting the pH by the addition of a pharmaceutically acceptable base. In preferred embodiments, the pharmaceutically acceptable acid is hydrochloric acid and the pharmaceutically acceptable base is sodium hydroxide.

The following description discloses that ropivacaine base is not hygroscopic and that it is much more stable than its hydrochloride or hydrochloride monohydrate salts. This novel invention is a significant improvement over the prior art in the elimination of the additional conventional manufacturing steps of preparing hydrochloride salts in order to provide an injectable solution. The conventional manufacturing steps are accompanied by a loss of yield and additional chemical waste generated in the process of making a hydrochloride salt as an intermediate. In addition, the novel method described herein also allows for better controls on the drug manufacturing process. For example, the aforementioned US 4,695,576 describes that 16 g of crude ropivacaine hydrochloride is converted to 14 g of pure ropivacaine hydrochloride (with a yield loss 12.5%) which is then converted to 12 g of ropivacaine hydrochloride monohydrate (with a yield loss of 18.3% on a molar basis); these steps, and hence these loses, are eliminated by the present invention.

The present invention in general provides process for preparing an aqueous solution of ropivacaine base comprising treating ropivacaine base in an aqueous media with an acid at a acid to base molar ratio greater than 1.0, and neutralizing with a base, providing a final injectable solution with a concentration of the ropivacaine base of from about 0.05% wt/vol to about 2.00% wt/vol, and more preferably from about 0.1% wt/vol to about 1.5% wt/vol. The osmolality is adjusted, if necessary, so that the final injectable solution has an osmolality preferably in the range of about 270 to 320 mOsM/kg to maintain the isotonicity of the injectable solution.

### Detailed Description of Specific Embodiments

The present invention describes a process of preparing an injectable aqueous pharmaceutical preparation of ropivacaine base. Ropivacaine base is incompletely soluble in aqueous solution containing hydronium ion up to equimolar concentrations in relation to the base. However, a solution of ropivacaine base can be prepared by dissolving the ropivacaine base in water containing an excess equivalent of acid and then neutralizing by the addition of a second base. Thus, for example, ropivacaine base can be dissolved in water containing 1.5 equivalents of hydrochloric acid and then neutralized by the addition of sodium hydroxide. Attempts to prepare an aqueous solution of ropivacaine base, even at a temperature of about 60 °C, in an aqueous media containing equimolar or less concentrated hydrochloric acid did not yield a completely homogeneous solution. The content of dissolved ropivacaine base in the solution is most preferably targeted to a base concentration of between 0.2% wt/vol to about 1.0% wt/vol, as shown in Tables1 and 2 of the examples.

As shown in certain examples below, ropivacaine base did not dissolve completely in equimolar acidic solutions. As shown, more than an equimolar concentration of hydronium ion is required to dissolve ropivacaine base in water or an aqueous medium. The excess hydronium ion, provided by hydrochloric acid in the examples, is neutralized with sodium hydroxide to provide a solution having a pharmaceutically acceptable pH, and the isotonicity, if required, is adjusted with sodium chloride. In this invention it is demonstrated by the examples that the solubility of ropivacaine base is enhanced by the presence of sodium chloride in an acidic pH solution, contrary to the normal theoretical concept of the common ion effect. This novel invention allows one to manufacture a stable isotonic aqueous ropivacaine formulation with fewer steps and less waste than shown in the art.

One embodiment of the process for preparing the injectable solution of ropivacaine base in aqueous medium comprises the following steps:
(1) Treating ropivacaine base or a suspension of ropivacaine base in aqueous medium with hydrochloric acid at a acid to base molar ratio of from about 1.1:1 to about 6.0:1 to yield a concentration of ropivacaine base in the range from about 0.1% wt/vol to about 1.5% wt/vol.
(2) Adjusting the pH of the solution to a range of from about 3.9 to about 6.5 using sodium hydroxide to obtain an isotonic solution.
(3) The preferred osmolality is in the range of about 270 to 320 mOsM/kg, and so optionally adding sodium chloride to adjust the osmolality.

In another embodiment is provided a process for preparing an injectable solution of ropivacaine base in aqueous medium via a concentrated intermediate solution, comprising the steps of:
(1) Treating ropivacaine base or a suspension of ropivacaine base in aqueous medium with hydrochloric acid at a acid to base molar ratio of about 1.1:1 to about 6.0:1 to yield a concentration of base in the range from about 1.5% wt/vol to about 30% wt/vol.
(2) Diluting the solution obtained by step (1) to a desired ropivacaine base concentration of about 0.1 % wt/vol to about 1.5% wt/vol with water or a sodium chloride solution.
(3) Adjusting the pH of the solution obtained in step (2) to be within the range of about 3.9 to about 6.5 using sodium hydroxide to obtain isotonicity of the solution measured as an osmolality in the range of about 270 to 320 mOsm/kg and optionally using sodium chloride in the adjustment of the isotonicity of the solution.

In another embodiment is provided a process for preparing an injectable solution of ropivacaine base in aqueous where a chloride ion is present initially in the medium, comprising the steps of:
(1) Treating ropivacaine base or a suspension of ropivacaine base in an aqueous medium with hydrochloric acid at a acid to base molar ratio greater than 1:1 to yield a concentration of ropivacaine base in the range from about 0.1 % wt/vol to about 1.5% wt/vol and a chloride concentration acceptable for an injectable solution.
(2) Adjusting the pH of the solution to be within the range of about 3.9 to about 6.5 directly to obtain an isotonic solution of osmolality in the range of about 270 to 320 mOsM/kg.

In this embodiment, the chloride concentration acceptable for an injectable solution is preferably about 0.3% wt/vol to about 0.7% wt/vol.

In still another embodiment is provided a process for preparing an aqueous solution of ropivacaine base by the steps of:
(1) Dissolving ropivacain base or a suspension of ropivacaine base at temperature above 60 °C in the presence of acid at greater than an equimolar concentration with respect to the base to obtain a stock solution.
(2) Diluting the stock solution obtained in step (1) to obtain the desired strength of ropivacaine in the solution.
(3) Preparing from the diluted stock solution an aqueous injectable solution of ropivacaine base with osmolality outside the range 270 to 320 mOsM/kg and adjusting the isotonicity by adding hydrochloric acid, sodium hydroxide, and/or sodium chloride to yield an osmolality of the solution in the range of about 270 to 320 mOsM/kg.

. The examples exemplify the use of sodium chloride for adjusting the chloride level and/or osmolality, although other water soluble chlorides, prefereably alkali and alkali earth metal chlorides, can be suitable.

The practice of this invention is illustrated by the following examples .

### Examples 1A to 1W

To separate suspensions of Ropivacaine base of purity 99.7% in 10 mL water were added 0.50 molar hydrochloric acid in molar ratios from 1:1 to about 3:1. Each resulting solution was warmed to about 50 °C and then cooled to room temperature with stirring for about 30 min., after which was added a suitable quantity of sodium chloride, and the total volume of each was made up to 25 mL. Osmolality of the filtered solution was determined using conventional freezing point osmometer. The pH of these solutions were in the range 3.2 to 3.5. The content of ropivacaine base dissolved in the solution was determined by HPLC against a control solution prepared by dissolving the same base in excess of hydrochloric acid. The results are furnished in Table 1, which shows the target (final) concentration of the ropivacaine base in solution, the amounts of the various components, and the properties of the solution.

**Table 1**

| Solubility of Ropivacaine base in hydrochloric acid solution | | | | | | | |
|---|---|---|---|---|---|---|---|
| # | Target conc. of base in solution, mg /mL | Ropivacaine base taken, mg, (mmol) | 0.5M Hydrochloric acid volume, mL (mmol) | NaCl added, mg | Molar ratio base:acid | Content of base determined by HPLC after filteration of the solution * | Osmolality of solution, mOsM/ kg |
| A | 2 | 50 (0.1825) | 0.365 (0.1825) | 0 | 1: 1 | 95.3 | 16 |
| B | 10 | 250 (0.912) | 1.825 (0.912) | 0 | 1: 1 | 81.6 | 71 |
| C | 2 | 50 (0.1825) | 0.365 (0.1825) | 214.2 | 1: 1 | 94.7 | 279 |
| D | 10 | 250 (0.912) | 1.825 (0.912) | 171.8 | 1: 1 | 91.7 | 293 |
| E | 2 | 50 (0.1825) | 0.402 (0.2001) | 0 | 1:1.1 | 87.4 | 12 |
| F | 10 | 250 (0.912) | 2.007 (1.003) | 0 | 1: 1.1 | 92.5 | 76 |
| G | 2 | 50 (0.1825) | 0.402 (0.2001) | 214.2 | 1: 1.1 | 98.0 | 291 |
| H | 10 | 250 (0.912) | 2.007 (1.003) | 166.0 | 1: 1.1 | 96.1 | 292 |
| J | 2 | 50 (0.1825) | 0.4568 (0.228) | 0 | 1: 1.25 | 99.6 | 19 |
| K | 10 | 250 (0.912) | 2.280 (1.140) | 0 | 1: 1.25 | 98.8 | 75 |
| L | 2 | 50 (0.1825) | 0.4568 (0.228) | 212.3 | 1: 1.25 | 97.9 | 294 |
| M | 10 | 250 (0.912) | 2.280 (1.140) | 158.3 | 1: 1.25 | 98.5 | 296 |
| N | N | 50 (0.1825) | 0.547 (0.274) | 0 | 1:1.5 | 100.5 | 23 |
| P | 10 | 250 (0.912) | 2.737 (1.368) | 0 | 1:1.5 | 98.6 | 107 |
| R | 2 | 50 (0.1825) | 0.547 (0.274) | 208.4 | 1:1.5 | 99.2 | 293 |
| S | 10 | 250 (0.912) | 2.737 (1.368) | 144.7 | 1:1.5 | 99.4 | 300 |
| T | 2 | 50 (0.1825) | 1.094 (0.547) | 0 | 1:3 | 100.1 | 42 |
| U | 10 | 250 (0.912) | 5.474 (2.736) | 0 | 1:3 | 99.3 | 78 |
| V | 2 | 50 (0.1825) | 1.094 (0.547) | 193.0 | 1:3 | 100.2 | 302 |
| W | 10 | 250 (0.912) | 5.474 (2.736) | 65.6 | 1:3 | 99.5 | 297 |

| | | | | | | | |
|---|---|---|---|---|---|---|---|
| * Value below 97% is considered incomplete solution. | | | | | | | |

### Examples 2A to 2W

To separate suspensions of Ropivacaine base of purity 99.7% in 10 mL water were added 0.50 molar hydrochloric acid in molar ratios of 1:1 to about 3:1. Each resulting solution was warmed to about 50 °C with stirring for about 30 min., then cooled to room temperature, after which was added a suitable quantity of sodium chloride. The pH was then adjusted to 5.0 using sodium hydroxide solution, and then the volume was made up to 25 mL. The osmolality of the filtered solution was determined using conventional freezing point osmometer. The content of ropivacaine base dissolved in the solution was determined by HPLC against a control solution prepared by dissolving the same base in excess of hydrochloric acid. The results are furnished in Table 2, analogous to those shown in Table 1.

**Table 2**

| Solubility of Ropivacaine base in solution after adjusting the pH to 5.0 | | | | | | | |
|---|---|---|---|---|---|---|---|
| # | Target conc. of base in solution, mg / mL | Ropivacaine base taken, mg, (mmol) | 0.5M Hydrochloric acid volume, mL (mmol) | Sodium chloride added, mg | Molar ratio base:acid | Content of base determined by HPLC after filteration of the solution * | Osmolality of solution, mOsM/ kg |
| A | 2 | 50 (0.1825) | 0.365 (0.1825) | 0 | 1: 1 | 88.6 | 17 |
| B | 10 | 250 (0.912) | 1.825 (0.912) | 0 | 1: 1 | 80.2 | 70 |
| C | 2 | 50 (0.1825) | 0.365 (0.1825) | 214.2 | 1: 1 | 93.3 | 294 |
| D | 10 | 250 (0.912) | 1.825 (0.912) | 171.8 | 1: 1 | 96.0 | 298 |
| E | 2 | 50 (0.1825) | 0.402 (0.2001) | 0 | 1: 1.1 | 97.0 | 20 |
| F | 10 | 250 (0.912) | 2.007 (1.003) | 0 | 1: 1.1 | 99.3 | 77 |
| G | 2 | 50 (0.1825) | 0.402 (0.2001) | 214.2 | 1: 1.1 | 98.5 | 298 |
| H | 10 | 250 (0.912) | 2.007 (1.003) | 166.0 | 1: 1.1 | 99.7 | 294 |
| J | 2 | 50 (0.1825) | 0.4568 (0.228) | 0 | 1: 1.25 | 95.8 | 20 |
| K | 10 | 250 (0.912) | 2.280 (1.140) | 0 | 1: 1.25 | 98.4 | 91 |
| L | 2 | 50 (0.1825) | 0.4568 (0.228) | 212.3 | 1: 1.25 | 99.3 | 292 |
| M | 10 | 250 (0.912) | 2.280 (1.140) | 158.3 | 1: 1.25 | 99.4 | 277 |
| N | 2 | 50 (0.1825) | 0.547 (0.274) | 0 | 1:1.5 | 99.7 | 24 |
| P | 10 | 250 (0.912) | 2.737 (1.368) | 0 | 1:1.5 | 96.6 | 105 |
| R | 2 | 50 (0.1825) | 0.547 (0.274) | 208.4 | 1:1.5 | 99.0 | 303 |
| S | 10 | 250 (0.912) | 2.737 (1.368) | 144.7 | 1:1.5 | 98.6 | 297 |
| T | 2 | 50 (0.1825) | 1.094 (0.547) | 0 | 1:3 | 100.1 | 130 |
| U | 10 | 250 (0.912) | 5.474 (2.736) | 0 | 1:3 | 99.5 | 130 |
| V | V | 50 (0.1825) | 1.094 (0.547) | 193.0 | 1:3 | 99.5 | 282 |
| W | W | 250 (0.912) | 5.474 (2.736) | 65.6 | 1:3 | 100.2 | 289 |

| | | | | | | | |
|---|---|---|---|---|---|---|---|
| * Value below 97% is considered incomplete solution. | | | | | | | |

### Example 3 Preparation with addition of sodium chloride

To a suspension of 2.0 g (7.29 mmol) ropivacaine base in 100 mL sterile water was added 43.76 mL of 0.5 molar (21.87 mmol) hydrochloric acid at a temperature around 25 °C with stirring for about 30 min. The pH of the solution was adjusted to about 5.0 using sodium hydroxide solution. Then 7.72 g of sodium chloride was added and the solution was made up to 1000 mL. The osmolality of the solution was determined to be 291 mOsM/kg.

### Example 4 Preparation with addition of sodium chloride

To a suspension of 2.0 g (7.29 mmol) ropivacaine base in 100 mL sterile water was added 2.188 mL of 10 molar (21.87 mmol) hydrochloric acid at a temperature around 25 °C with stirring for about 30 min. The pH of the solution was adjusted to about 5.0 using sodium hydroxide solution, then 7.72 g sodium chloride was added, and solution was then made up to 1000 mL. The osmolality of the solution was determined to be 290 mOsM/kg.

### Example 5 Preparation with addition of sodium chloride (scale up)

To a suspension of 10 g (36.45 mmol) ropivacaine base in 100 mL sterile water was added 218.8 mL of 0.5 molar (109.35 mmol) hydrochloric acid at a temperature around 25 °C with stirring for about 30 min. The pH of the solution was adjusted to about 5.0 using sodium hydroxide solution, after which was added 2.624 g sodium chloride, and then the solution was made up to 1000 mL. The osmolality of the solution was determined to be 286 mOsM/kg.

### Example 6 Preparation with addition of sodium chloride (scale up)

To a suspension of 10 g (36.45 mmol) ropivacaine base in 100 mL sterile water was added 10.94 mL of 10 molar (109.35 mmol) hydrochloric acid at a temperature around 25 °C with stirring for about 30 min.; the pH of the solution was adjusted to about 5.0 using sodium hydroxide solution; 2.624 g sodium chloride was added; and the solution was made up to 1000 mL. The osmolality of the solution was determined to be 288 mOsM/kg.

### Example 7 Preparation of injectable concentration by dilution

To a suspension of 40 g (145.8 mmol) ropivacaine base in 200 mL sterile water was added 43.74 mL of 10 molar (437.4 mmol) hydrochloric acid at a temperature around 25 °C with stirring for about 30 min. This solution was diluted with sterile water to a total volume of 1000 mL to make a stock solution. 125 mL of this stock solution was diluted to 500 mL with sterile water to achieve a concentration of 10 mg / mL ropivacaine in solution, then the pH was adjusted to 5.0 using sodium hydroxide, after which was added about 1.309 g sodium chloride to adjust the osmolality to 290.

### Example 8 Preparation of injectable concentration by dilution

To a suspension of 40 g (145.8 mmol) ropivacaine base in 200 mL sterile water was added 43.74 mL of 10 molar (437.4 mmol) hydrochloric acid at a temperature around 25 °C with stirring for about 30 min. This solution was made up to 1000 mL with sterile water to make a stock solution. 50 mL of this stock solution was diluted to 1000 mL with sterile water to achieve a concentration of 2 mg / mL ropivacaine, then the pH was adjusted to 5.0 using sodium hydroxide, and thereafter about 7.734 g sodium chloride was added to adjust the osmolality to 290.

### Example 9 Without addition of sodium chloride

To a suspension of 2.0 g (7.29 mmol) ropivacaine base in 100 mL sterile water was added 308.4 mL of 0.5 molar (308.37 mmol) hydrochloric acid at a temperature around 25 °C with stirring for about 30 min. The pH of the solution was adjusted to about 5.0 using sodium hydroxide solution and the resulting solution was made up to 1000 mL to produce a stock solution. The osmolality of the stock solution was determined to be 286 mOsM/kg.

### Example 10 Without addition of sodium chloride

To a suspension of 2.0 g (7.29 mmol) ropivacaine base in 100 mL sterile water was added 15.42 mL of 10 molar (308.37 mmol) hydrochloric acid at a temperature around 25 °C with stirring for about 30 min. The pH of this solution was adjusted to about 5.0 using sodium hydroxide solution and the resulting solution was made up to 1000 mL to produce a stock solution. The osmolality of the stock solution was 285 mOsM/kg.

### Example 11 Without addition of sodium chloride

To a suspension of 10 g (36.45 mmol) ropivacaine base in 200 mL sterile water was added 308.4 mL of 0.5 molar (154 mmol) hydrochloric acid at a temperature around 25 °C with stirring for about 30 min.; the pH of the solution was adjusted to about 5.0 using sodium hydroxide solution, and then diluted to make up to1000 mL. The osmolality of the resulting solution was 292 mOsM/kg.

### Example 12 Preparation with addition of sodium chloride

To a suspension of 2.0 g (7.29 mmol) ropivacaine base was added 100 mL sterile water containing 7.72 g sodium chloride, then 43.76 mL of 0.5 molar (21.87 mmol) hydrochloric acid at temperature around 25 °C was added with stirring for about 30 min. The pH of the solution was adjusted to about 5.0 using sodium hydroxide solution and solution was made up to 1000 mL. Osmolality of the solution was determined to be 293 mOsM/kg.

### Example 13 Preparation with addition of sodium chloride

To a suspension of 2.0 g (7.29 mmol) ropivacaine base was added 100 mL sterile water containing 8.56 g sodium chloride; then 14.6 mL of 0.5 molar (7.29 mmol) hydrochloric acid heated to a temperature of about 80°C was added with stirring for about 45 min. The solution was cooled to about 25 °C. The pH of the solution wass adjusted to about 5.0 using sodium hydroxide solution and solution was made up to 1000 mL. The content of dissolved ropivacaine was found to be 97.3%, and the osmolality of the solution was determined to be 291 OsM/kg.

### Example 14

To a suspension of 10 g (36.45 mmol) Ropivacaine base was added 200 mL sterile water containing 6.87 g sodium chloride; thereafter, 73.0 mL of 0.5 molar (36.45 mmol) hydrochloric acid heated to a temperature of about 80° C was added with stirring for about 45 min. The solution was cooled to about 25 °C and the pH of the solution was adjusted to about 5.0 using sodium hydroxide solution; the solution was then made up to 1000 mL. The content of dissolved ropivacaine was found to be 99.0%, and the osmolality of the solution was found to be 297 mOsM/kg.

Of course, the concentration of L-N-n-propylpipecolic acid-2,6-xylidide when administered a s a local anesthetic will be regulated to avoid tissue irritation and toxic reaction effects. The regulation of the concentration of this anesthetic can be achieved by following conventional toxicity tests and protocols heretofore established for local anesthetics.

## Claims

1. A process for making a solution of ropivacaine base in an aqueous medium comprising:
(1) providing ropivacaine base or a suspension of ropivacaine base in an aqueous medium;
(2) mixing said ropivacaine base or suspension with hydrochloric acid wherein the acid is present in an extramolar amount effective to dissolve said ropivacaine; and
(3) adjusting the pH of the solution with sodium hydroxide to a range of about 3.9 to about 6.5.

2. The process of claim 1, wherein the content of ropivacaine base after step (2) is between about 0.05% wt/vol and about 7.0 % wt/vol.

3. The process of claim 1, further comprising the step of adjusting the osmolality of the solution by the addition of a salt.

4. The process of claim 3, wherein the osmolality is adjusted to be between about 270 mOsM/kg to about 320 mOsM/kg.

5. The process of claim 1, further comprising diluting the solution obtained after step (2) to contain about 1.5% wt/vol to about 4.5% wt/vol of ropivacaine base.

6. The process of claim 1, wherein the molar ratio of ropivacaine base to acid after step (2) is at least about 1 to 1.1.

7. The process of claim 6, wherein the molar ratio of ropivacaine base to acid after step (2) is at least about 1 to 1.5.

8. The process of claim 7, wherein the molar ratio of ropivacaine base to acid after step (2) is at about 1 to 3.

9. The process of claim 1 or claim 3, wherein the chloride concentration is in the range of from about 0.3% wt/vol to about 0.7% wt/vol.

10. The process of claim 1, wherein the pH is about 5.

## Patentansprüche

1. Verfahren zur Herstellung einer Lösung von Ropivacain-Base in einem wässrigen Medium, umfassend:
(1) Bereitstellen von Ropivacain-Base oder einer Suspension von Ropivacain-Base in einem wässrigen Medium;
(2) Mischen der Ropivacain-Base oder-Suspension mit Chlorwasserstoffsäure, wobei die Säure in einer mehr als molaren Menge vorliegt, die wirksam ist, um das Ropivacain zu lösen; und
(3) Einstellen des pH der Lösung mit Natriumhydroxid auf einen Bereich von etwa 3,9 bis etwa 6,5.

2. Verfahren nach Anspruch 1, bei dem der Gehalt an Ropivacain-Base nach Schritt (2) zwischen etwa 0,05 Gew.-%/Vol. und etwa 7,0 Gew.-%/Vol. liegt.

3. Verfahren nach Anspruch 1, weiter umfassend den Schritt der Einstellung der Osmolalität der Lösung durch Zugabe eines Salzes.

4. Verfahren nach Anspruch 3, bei dem die Osmolalität auf zwischen etwa 270 mOsM/kg bis etwa 320 mOsM/kg eingestellt wird.

5. Verfahren nach Anspruch 1, weiter umfassend das Verdünnen der nach Schritt (2) erhaltenen Lösung, so dass sie etwa 1,5 Gew.-%/Vol. bis etwa 4,5 Gew.-%/Vol. Ropivacain-Base enthält.

6. Verfahren nach Anspruch 1, bei dem das Molverhältnis von Ropivacain-Base zur Säure nach Schritt (2) mindestens etwa 1 zu 1,1 beträgt.

7. Verfahren nach Anspruch 6, bei dem das Molverhältnis von Ropivacain-Base zur Säure nach Schritt (2) mindestens etwa 1 zu 1,5 beträgt.

8. Verfahren nach Anspruch 7, bei dem das Molverhältnis von Ropivacain-Base zu Säure nach Schritt (2) etwa 1 zu 3 beträgt.

9. Verfahren nach Anspruch 1 oder Anspruch 3, bei dem die Chlorid-Konzentration im Bereich von etwa 0,3 Gew.-%/Vol. bis etwa 0,7 Gew.-%/Vol. liegt.

10. Verfahren nach Anspruch 1, bei dem der pH etwa 5 beträgt.

## Revendications

1. Procédé de préparation d'une solution de ropivacaïne base dans un milieu aqueux comprenant les étapes consistant à :
(1) fournir de la ropivacaïne base ou une suspension de ropivacaïne base dans un milieu aqueux ;
(2) mélanger ladite ropivacaïne base ou suspension avec de l'acide chlorhydrique où l'acide est présent dans une quantité extramolaire efficace pour dissoudre ladite ropivacaïne ; et
(3) ajuster le pH de la solution avec de l'hydroxyde de sodium dans une gamme d'environ 3,9 à environ 6,5.

2. Procédé selon la revendication 1, dans lequel la teneur en ropivacaïne base après l'étape (2) est comprise entre environ 0,05 % en poids par volume et environ 7,0 % en poids par volume.

3. Procédé selon la revendication 1, comprenant en outre l'étape d'ajustement de l'osmolalité de la solution par l'addition d'un sel.

4. Procédé selon la revendication 3, dans lequel l'osmolalité est ajustée à une valeur comprise entre environ 270 mOsM/kg à environ 320 mOsM/kg.

5. Procédé selon la revendication 1, comprenant en outre la dilution de la solution obtenue après l'étape (2) pour qu'elle contienne environ 1,5 % en poids par volume à environ 4,5 % en poids par volume de ropivacaïne base.

6. Procédé selon la revendication 1, dans lequel le rapport molaire entre ropivacaïne base et l'acide après l'étape (2) est au moins d'environ 1 à 1,1.

7. Procédé selon la revendication 6, dans lequel le rapport molaire entre ropivacaïne base et l'acide après l'étape (2) est au moins d'environ 1 à 1,5.

8. Procédé selon la revendication 7, dans lequel le rapport molaire entre ropivacaïne base et l'acide après l'étape (2) est au moins d'environ 1 à 3.

9. Procédé selon la revendication 1 ou la revendication 3, dans lequel la concentration en chlorure est dans la gamme d'environ 0,3 % en poids par volume à environ 0,7 % en poids par volume.

10. Procédé selon la revendication 1, dans lequel le pH est d'environ 5.
